(19) Europäisches Patentamt
European Patent Office
Office européen des brevets

(11) **EP 4 329 523 B1**

(12) **EUROPÄISCHE PATENTSCHRIFT**

(45) Veröffentlichungstag und Bekanntmachung des Hinweises auf die Patenterteilung:
**22.10.2025 Patentblatt 2025/43**

(21) Anmeldenummer: **22725790.4**

(22) Anmeldetag: **26.04.2022**

(51) Internationale Patentklassifikation (IPC):
*A24B 15/12* (2006.01)   *A24C 5/34* (2006.01)
*G01N 22/04* (2006.01)   *A24B 15/28* (2006.01)
*A24B 15/32* (2006.01)

(52) Gemeinsame Patentklassifikation (CPC):
**G01N 22/04; A24B 15/12; A24B 15/282;**
**A24B 15/30;** A24C 5/3412; G01N 2223/621;
G01N 2223/622; G01N 2223/642; G01N 2223/645

(86) Internationale Anmeldenummer:
**PCT/EP2022/061054**

(87) Internationale Veröffentlichungsnummer:
**WO 2022/229186 (03.11.2022 Gazette 2022/44)**

(54) **VERFAHREN ZUR BESTIMMUNG EINES ZUSATZSTOFFGEHALTS IN EINEM TABAKPAPIER FÜR ELEKTRISCHE ZIGARETTEN**

METHOD FOR DETERMINING AN ADDITIVE CONTENT IN A TOBACCO PAPER FOR ELECTRIC CIGARETTES

PROCÉDÉ DE DÉTERMINATION D'UNE TENEUR EN ADDITIFS DANS UN PAPIER ENVELOPPE POUR TABAC POUR CIGARETTES ÉLECTRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.04.2021 DE 102021110760**

(43) Veröffentlichungstag der Anmeldung:
**06.03.2024 Patentblatt 2024/10**

(73) Patentinhaber: **TEWS Elektronik GmbH & Co. KG 22459 Hamburg (DE)**

(72) Erfinder:
• **SCHLEMM, Udo**
  **22607 Hamburg (DE)**
• **RICHTER, Hendrik**
  **20255 Hamburg (DE)**

(74) Vertreter: **Hauck Patent- und Rechtsanwälte PartmbB**
**Postfach 11 31 53**
**20431 Hamburg (DE)**

(56) Entgegenhaltungen:
EP-A1- 2 587 253      WO-A1-2017/080982
WO-A1-2021/033637    DE-A1- 102009 004 457
DE-A1- 102012 209 954   US-A1- 2022 160 027

**Beschreibung**

Die vorliegende Erfindung betrifft ein Verfahren zur Messung eines Zusatzstoffgehalts in einem Tabakpapier für elektrische Zigaretten.

Glycerin (E422) ist ein Zusatzstoff, der als Feuchthaltemittel für Tabakwaren verwendet wird. In Zigaretten- und Pfeifentabak soll das Feuchthaltemittel vor allem die Lagerungszeit des Produkts verlängern und sein Austrocknen verhindern. Bei Shisha-Tabak werden höhere Mengen an Feuchthaltemittel dem Tabak zugemischt, um einerseits die Verbrennung des Tabaks zu verhindern und andererseits einen möglichst dichten Dampf zu erzeugen. Weiterhin findet Glycerin Verwendung als Nebelfluid in elektrischen Zigaretten, wo es unter Hitzeeinwirkung einen weißen dichten Dampf bildet.

Bei der Herstellung einer bestimmten Art von elektrischen Zigaretten werden Glycerin, Bindemittel, Aromastoffe und Tabak sowie weitere aerosolbildende Zusatzstoffe zu einem Brei gemischt. Dieser Brei wird gewalzt und getrocknet, wodurch ein sogenanntes Tabakpapier entsteht. Dieses Tabakpapier wird nachfolgend gekräuselt, beispielsweise in einem Crimper und zu einem Strang verarbeitet. Bei der Herstellung des Breies wird ein definierter Anteil Glycerin zugesetzt. Ein undefinierter Anteil hiervon kann jedoch im Trocknungsprozess verlorengehen. Eine nachträgliche Kontrolle des Glyceringehalts ist daher für die Qualitätssicherung des Endprodukts sehr wichtig.

Aus DE 10 2007 041 429 A1 ist ein Verfahren zur Messung eines Feuchtewerts F von dielektrischen Stoffen unter Verwendung von mindestens einem Mikrowellenresonator bekannt, wobei für mindestens zwei Resonanzmodi mit jeweils voneinander verschiedenen Resonanzfrequenzen jeweils eine Verschiebung der Resonanzfrequenz A ausgewertet und aus den gemessenen Verschiebungen der Resonanzfrequenz ein dichteunabhängiger Feuchtewert berechnet wird. Es ist bekannt, die Resonanzfrequenzverschiebungen für weit auseinanderliegende Resonanzfrequenzen zu messen. Dabei wird bei einer Resonanzfrequenz unterhalb von 1 GHz gemessen, während die andere Resonanzfrequenzverschiebung bei einer Frequenz oberhalb von 7 GHz gemessen wird.

Aus WO 2017/080982 A1 ist eine Vorrichtung und ein Verfahren zur Bestimmung des Anteils mindestens eines Zusatzstoffes in einem tabakhaltigen Stoff bekannt geworden. Um die Anteile von Tabak und Wasser zu bestimmen, werden zwei Messgrößen aus einem elektromagnetischen Wechselfeld gewonnen. Es wird ferner erklärt, dass, um den Anteil mindestens eines weiteren Zusatzstoffes zu bestimmen, eine weitere Messgröße durch Messung mit einem zweiten elektromagnetischen Wechselfeld bei einer zweiten Messfrequenz gewonnen werden muss. Indem die Messgrößen, beispielsweise Betrag und Phase der elektromagnetischen Wechselfelder oder Resonanzverschiebung und Resonanzverbreiterung bei zwei unterschiedlichen Messfrequenzen unabhängig voneinander gemessen werden, stehen insgesamt vier Messgrößen zur Verfügung, aus denen Rückschlüsse über die Gewichtsanteile von Tabak, Wasser und dem mindestens einen Zusatzstoff möglich ist. Es wird ferner darauf hingewiesen, dass zur Bestimmung von Tabak, Wasser und einem Zusatzstoff grundsätzlich drei Messgrößen ausreichen, jedoch vier Messgrößen die Genauigkeit der Messung weiter erhöhen. Dieser aus dem Stand der Technik bekannte Ansatz besteht darin, die Feuchte und die Gewichtsanteile des Tabaks und des Zusatzstoffes aus den mindestens vier ermittelten Messgrößen in einer Datenverarbeitungsvorrichtung als beste Lösung eines überbestimmten Gleichungssystems, beispielsweise mit minimalen Fehlerquadraten, vorteilhaft zu bestimmen. Die Gewichtsanteile von Zusatzstoffen können dabei zusätzlich zu der Feuchte und dem Gewichtsanteil des Tabaks bestimmt werden.

Der Erfindung liegt die Aufgabe zugrunde, ein Verfahren zur Messung des Zusatzstoffgehalts in einem Tabakpapier bereitzustellen, das mit einfachen Mitteln möglichst genaue Werte liefert.

Erfindungsgemäß wird die Aufgabe durch das Verfahren mit den Merkmalen aus Anspruch 1 gelöst. Vorteilhafte Ausgestaltungen bilden die Gegenstände der Unteransprüche.

Das erfindungsgemäße Verfahren ist vorgesehen, um den Zusatzstoffgehalt in einem Tabakpapier für elektrische Zigaretten möglichst genau zu messen. Der Zusatzstoffgehalt wird in Volumen-% oder in Gewicht-% bestimmt. Das Tabakpapier wird aus einer breiigen Masse von Zusatzstoffen, Wasser, Aromastoffen und Tabak hergestellt, wobei die breiige Masse bevorzugt nach einem Walzschritt zu einem einlagigen Tabakpapier getrocknet wird. Bei den Trocknungsvorgang entweicht eine nicht vorhersehbare Menge an Zusatzstoffen und Wasser, so dass eine Messung zur Bestimmung des Zusatzstoffgehalts an dem Tabakpapier zu erfolgen hat. Die Messung erfolgt mit mindestens einem Mikrowellenresonator mit zwei Resonanzmodi mit zwei unterschiedlichen Resonanzfrequenzen. Die kleinere der beiden Resonanzfrequenzen liegt in einem Frequenzbereich von weniger als 1 GHz, die größere der beiden Resonanzfrequenzen besitzt Werte von mehr als 2 GHz, wobei der untere Bereich in einem unteren Mikrowellenbereich von 800 MHz liegen kann. Für jede der beiden Resonanzfrequenzen wird jeweils ein dichteunabhängiger Feuchtewert berechnet, wobei der Zusatzstoffgehalt abhängig von den beiden dichteunabhängigen Feuchtewerten bestimmt wird. Der dichtunabhängige Feuchtewert ist bevorzugt jeweils ein dichteunabhängiger Feuchtewinkel. Der jeweilige dichteunabhängige Feuchtewert ist dadurch gekennzeichnet, dass er unabhängig von der Dichte ist und indikativ für eine Feuchte in dem Messgut. Anders als im Stand der Technik werden nicht vier Messwerte zu einem Gleichungssystem zusammengesetzt, sondern die durch die beiden Resonanzmodi gewonnenen Werte werden zu dichteunabhängigen Feuchtewerten verarbeitet. Indem der Zusatzstoffgehalt unabhängig von dem Tabakgehalt über eine dichteunabhängige Größe wie beispielsweise den Feuchtewinkel bestimmt wird, können erheblich genauere Werte erzielt werden. Physikalisch formuliert bedeutet dies, dass die Auswertung von Zustandsgrößen, die sich als intensive Zustandsgrößen darstellen, deutlich bessere Ergebnisse liefert als die Auswertung von extensiven Zustandsgrößen.

Bevorzugt wird als Zusatzstoff in der tabakverarbeitenden Industrie Glycerin eingesetzt. Gerade bei Glycerin liefern die dichteunabhängigen Feuchtewerte sehr genaue Ergebnisse.

In einer bevorzugten Ausgestaltung wird der Glyceringehalt g linear von beiden Feuchtewinkeln und einem Offsetwert bestimmt. Es ist für den Glyceringehalt g wichtig, dass beide Feuchtewerte, also sowohl von der Resonanzfrequenz aus dem Hochfrequenzbereich als auch von der Resonanzfrequenz im Mikrowellenbereich, zu dem Glyceringehalt beitragen.

In einer ebenfalls bevorzugten Weiterentwicklung wird eine Feuchte für das Tabakpapier, abhängig von dem Feuchtewert bei der größeren Frequenz gemessen. Es ist wichtig, einzusehen, dass die Messung der Feuchte des Tabakpapiers nur von dem dichteunabhängigen Feuchtewert bei der höheren Frequenz abhängt und etwaige Beiträge von der niedrigeren Resonanzfrequenz vernachlässigt werden können. Dieses Verhalten zeigt gerade die Messung des Glyceringehalts nicht, da diese von beiden Feuchtewerte abhängt.

In einer bevorzugten Weiterbildung ist der mindestens eine Mikrowellenresonator als ein planarer Sensor ausgebildet. Der planare Sensor besitzt ein aus dem Resonatorkörper austretendes Feld, das mit dem Messobjekt wechselwirkt. Beim Einsatz eines planaren Sensors wird das Tabakpapier über eine ebene Sensorfläche gefiltert wird und dabei durch ein Messfeld transportiert.

Neben planaren Sensoren können grundsätzlich auch Spaltsensoren vorgesehen sein, bei denen das Tabakpapier über einen Spalt durch einen Resonatorhohlraum transportiert wird.

In einer möglichen Ausgestaltung kann die Messung an dem einlagigen Tabakpapier direkt erfolgen. Es hat sich jedoch herausgestellt, dass die Messung auch an dem zu einer Bobine aufgewickelten Tabakpapier erfolgen kann. Grundsätzlich ist auch möglich, beide Messungen nacheinander vorzunehmen. Alternativ oder zusätzlich kann die Messung auch in oder direkt nach dem Trocknungsschritt erfolgen. Die Messung kann dabei nach Abschluss des Trocknungsvorgangs erfolgen oder auch zu einem definierten Zeitpunkt während des Trocknungsvorgangs.

In einer bevorzugten Ausgestaltung ist es möglich, den Glyceringehalt des Tabakpapiers vor seiner Weiterverarbeitung zu einem Strang, also bevor es in den Crimper eintritt, zu messen.

Das erfindungsgemäße Messverfahren ist sehr zuverlässig und kann die Zugabe von Wasser und/oder Glycerin zu der breiigen Masse abhängig von dem gemessenen Feuchtewerten steuern. Auf diese Weise kann ein gewünschter Wert für den Glycerin- und Feuchtegehalt eingeregelt werden.

In einer bevorzugten Weiterbildung ist der Feuchtewert ein Feuchtewinkel. Der Feuchtewinkel wird als Quotient aus Resonanzfrequenzverschiebung und der Verbreiterung der Halbwertsbreite bestimmt. Bei der Resonanzfrequenzverschiebung werden die Änderungen der Frequenz in Hertz zwischen leerem und befülltem Resonator miteinander verglichen. Ebenfalls wird die Halbwertsbreite der Resonanzkurve bei unbefülltem Resonator betrachtet. Statt der Halbwertsbreite ist es auch möglich, andere durch die Dämpfung der Resonanz hervorgerufene Größen, wie beispielsweise die Amplitude der Resonanzkurve, zu betrachten. Es hat sich als vorteilhaft herausgestellt, den Feuchtewinkel als Arcus-Tangens des dichteunabhängigen Quotienten aus Resonanzfrequenzverschiebung und der Verbreiterung der Halbwertsbreite zu bestimmen.

Das erfindungsgemäße Verfahren wird nachfolgend an einem Ausführungsbeispiel näher erläutert. Es zeigen:

Figur 1    in einer schematischen Ansicht Orte für die Glycerinmessung in der Primary,

Figur 2    mögliche Orte für die Glycerinmessung im Crimper,

Figur 3    die Messwerte für den Feuchtewinkel zweier Moden in Abhängigkeit von Feuchte- und Glyceringehalt, und

Figur 4    Ergebnisse der Glycerinmessung.

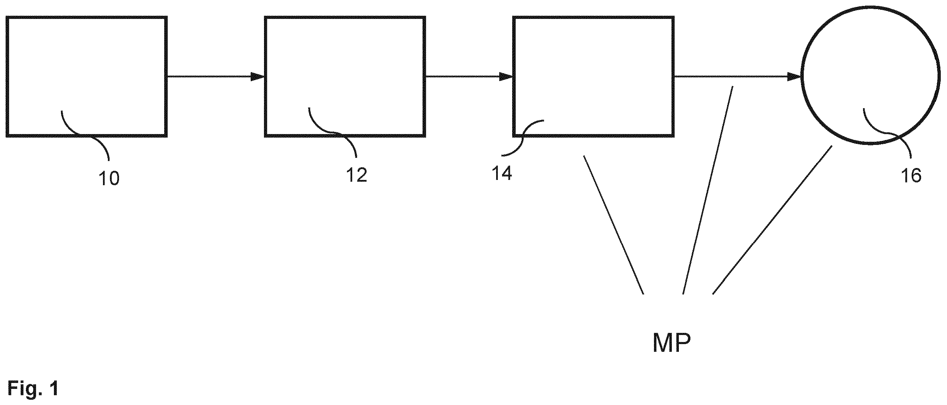

Figure 1 zeigt in einer sehr schematischen Ansicht einen Mischer 10, in dem zur Verwendung bei elektrischen Zigaretten Glycerin mit Wasser, Bindemittel, Aromastoffe, weitere aerosolbildende Zusatzstoffe und Tabak zu einem Brei gemischt werden. Das Ausführungsbeispiel stellt auf Glycerin ab, es können aber auch andere Zusatzstoffe mit oder ohne Glycerin in gleicher Weise eingesetzt und gemessen werden. Hierbei werden die Zutaten in genau definierten Verhältnissen zugesetzt und in dem Mischer zu einem homogenen Gemenge in Form eines Breis verarbeitet. Der so gebildete Brei wird in einer Walzmaschine 12 gewalzt und als Flachmaterial dem Trockner 14 zugeführt. In dem Trockner 14 erfolgt der Trocknungsprozess, bei dem eine undefinierte Menge an Wasser und Glycerin aus dem Tabakpapier verloren geht. Das getrocknete Tabakpapier wird in 16 zu einer Bobine aufgerollt. Mögliche Messplätze MP, um die Feuchtigkeit zu kontrollieren und den Glyceringehalt zu messen, liegen beispielsweise im Trockner 14, auf der Strecke des getrockneten Tabakpapiers von dem Trockner 14 zu der Bobine 16 und direkt an der Bobine 16. Die gewonnenen Werte für den Glyceringehalt und/oder den Feuchtegehalt können an den Mischer 10 zurückgemeldet werden, um den Glyceringehalt auf einen gewünschten Wert einzuregeln. Auch ist eine Reglung des Feuchtegehalts möglich. Ebenso können die gemessenen Werte auch herangezogen werden, um die Parameter des Trocknungsprozesses an die gewünschten Werte für Feuchte und Glycerin anzupassen.

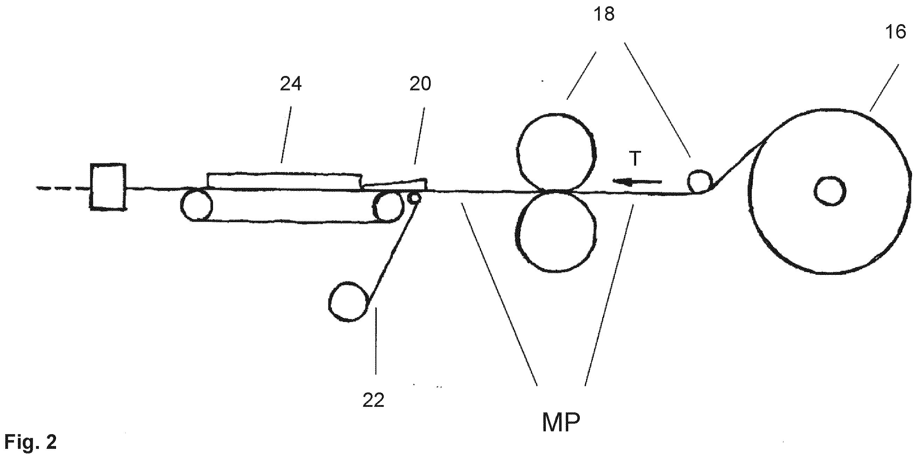

Figur 2 zeigt in einer schematischen Ansicht, wie Tabakpapier 18 von der Bobine 16 entlang der Transportrichtung T abgewickelt wird. Über Transportwalzen 18 wird das Tabakpapier einem Crimper 20 zugeführt. Dem Crimper 20 wird ebenfalls Papier 22 zur Strangumhüllung zugeführt, um im Bereich 24 zu einem Strang geformt zu werden. Die möglichen Messplätze MP liegen entlang der Strecke von der Bobine zu dem Crimper 20, bei dem weiteres Papier zur Strangbildung zugeführt wird.

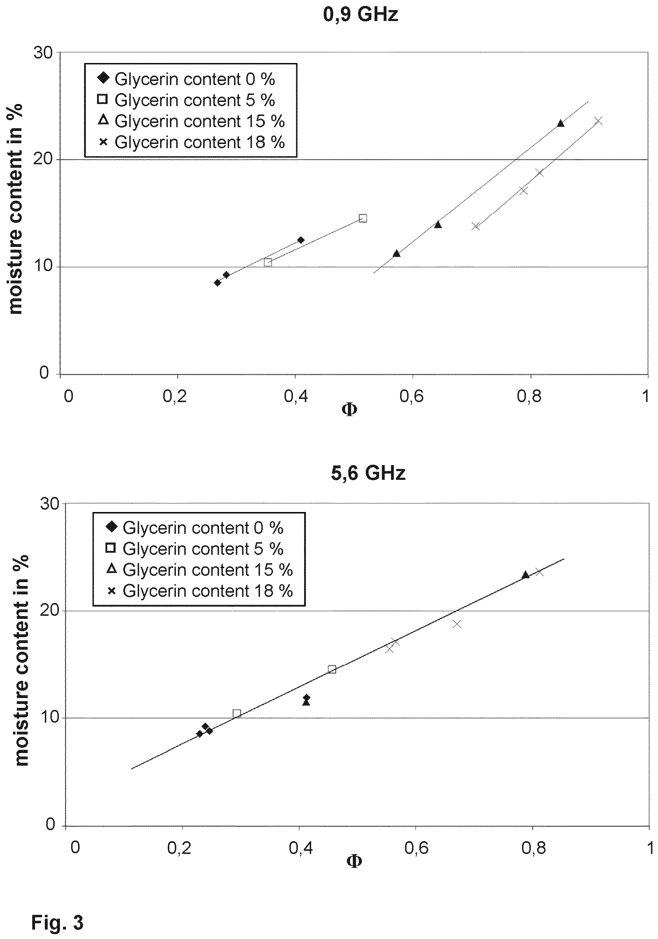

Figur 3 zeigt für zwei Frequenzen mit 0,9 GHz und 5,6 GHz die aufgetragenen Feuchtewerte (moisture content in %) über dem Feuchtewinkel $\Phi$. Der Feuchtewinkel $\Phi$ wird gebildet als Arcus-Tangens des Quotienten von B/A, wobei A die Resonanzfrequenzverschiebung und B die Verbreiterung der Resonanzkurve beschreibt.

Die Messungen wurden bei unterschiedlichem Glyceringehalt und unterschiedlichem Feuchtegehalt durchgeführt. Die zweite Messung wurde am gleichen Material für die größere Frequenz aufgenommen.

Die Messung bei 5,6 GHz zeigt, dass die Feuchtewinkel $\Phi$ unabhängig vom Glyceringehalt der Proben sind und nur von deren Feuchtegehalt abhängen. Dies ergibt sich aus der Proportionalität von Feuchtewinkel und Feuchtegehalt bei unterschiedlichem Glyceringehalt. Deshalb kann die in der Figur 3 eingezeichnete Regressionsgerade als Kalibration für eine glycerinunanhängige Feuchtemessung verwendet werden.

Die Messung bei 0,9 GHz zeigt dagegen, dass die gemessenen Feuchtewinkel $\Phi$ vom Feuchte- und vom Glyceringehalt abhängen. Die Proben mit gleichem Glyceringehalt sind in der Figur durch gesonderte lineare Regressionen gekennzeichnet. Zur Kompensation des Einflusses der Variation der Materialfeuchte müssen zur Messung des Glyceringehaltes die Feuchtewinkel $\Phi$ beider Frequenzen berücksichtigt werden.

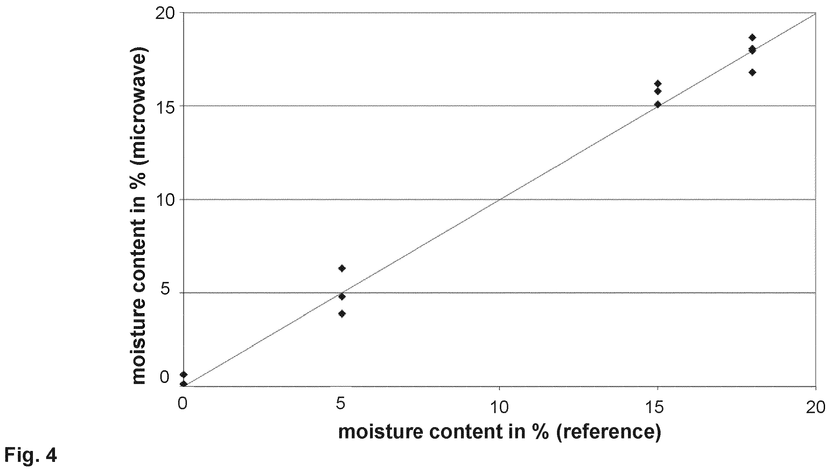

Figur 4 zeigt das Ergebnis der Auswertung, wobei der Referenzwert für den Glyceringehalt gegenüber dem gemessenen Glycerinwert aufgetragen wurde. Die Berechnung erfolgt unter Verwendung der Feuchtewinkel $\Phi$ beider Frequenzen in der im Folgenden aufgeführten Kalibrationsgleichung. Deutlich zu erkennen ist die gute Übereinstimmung der Werte mit der Ausgleichsgeraden, bei der die gemessenen Werte nur wenige Prozent von den Referenzwerten abweichen.

Für die Messung der Feuchte wird der Messwert der Resonanzmode mit der hohen Frequenz $\Phi_H$ genutzt. Ein Ansatz für den Feuchtewert u ist:

$$u = a_1 \cdot \Phi_H + a_2$$

wobei $a_1$, $a_2$ hier Kalibrationskoeffizienten darstellen. Sind die Kalibrationskoeffizienten bestimmt, so kann aus dem gemessenen Feuchtewinkel $\Phi_H$ direkt der Feuchtewert bestimmt werden.

Für die Bestimmung des Glyceringehalts wird der Feuchtewinkel beider Moden benutzt:

$$g = b_1 \cdot \Phi_L + b_2 \cdot \Phi_H + b_3$$

wobei $b_1$, $b_2$ und $b_3$ die Kalibrationskoeffizienten sind. Wichtig hierbei ist, dass beide Feuchtewinkel als masseunabhängige Größe in die Bestimmung des Glyceringehalts eingehen und so die Messung von Feuchte- und Glyceringehalt unabhängig von der Masse des Messproduktes ist. Der wie im Stand der Technik über ein eventuell überbestimmtes Gleichungssystem ermittelte Masseanteil des Messproduktes verschlechtert die Messgenauigkeit. Die zusätzliche Bestimmung des Anteils an Tabak kann bei dem erfindungsgemäßen Ansatz, der auf masseunabhängige Messgrößen abstellt, nicht erfolgen.

## Patentansprüche

1. Verfahren zur Messung des Zusatzstoffgehalts in einem Tabakpapier für elektrische Zigaretten, das aus einer breiigen Masse von Zusatzstoffen, Wasser, Aromastoffen und Tabak hergestellt wird, wobei die breiige Masse zu einem einlagigen Tabakpapier getrocknet wird, **dadurch gekennzeichnet, dass** das Tabakpapier mit mindestens einem Mikrowellenresonator mit zwei Resonanzmoden mit zwei Resonanzfrequenzen ($f_L$, $f_H$) gemessen wird, wobei die kleinere der beiden Resonanzfrequenzen ($f_L$) im Frequenzbereich mit weniger als 1 GHz und die größere der beiden Resonanzfrequenzen im Mikrowellenbereich mit mehr als 2 GHz liegt, für jede der beiden Resonanzmoden wird ein dichteunabhängiger Feuchtewert ($\Phi_{L,H}$) berechnet, der Glyceringehalt (g) wird abhängig von den beiden Feuchtewerten ($\Phi_{L,H}$) bestimmt.

2. Verfahren nach Anspruch 1, **dadurch gekennzeichnet, dass** der Zusatzstoff vollständig oder teilweise aus Glycerin besteht.

3. Verfahren nach Anspruch 1 oder 2, **dadurch gekennzeichnet, dass** der Zusatzstoffgehalt (g) linear von beiden dichteunabhängigen Feuchtewerten ($\Phi_{L,H}$) und einem Offset-Wert abhängt.

4. Verfahren nach einem der Ansprüche 1 bis 3, **dadurch gekennzeichnet, dass** eine Feuchte für das Tabakpapier abhängig von dem dichteunabhängigen Feuchtewert ($\Phi_H$) bei der größeren Frequenz gemessen wird.

**5.** Verfahren nach einem der Ansprüche 1 bis 4, **dadurch gekennzeichnet, dass** der Zusatzstoffgehalt (g) unabhängig von der Masse des Tabakpapiers bestimmt wird.

**6.** Verfahren nach einem der Ansprüche 1 bis 5, **dadurch gekennzeichnet, dass** der mindestens eine Mikrowellenresonator einen planaren Sensor aufweist.

**7.** Verfahren nach einem der Ansprüche 1 bis 6, **dadurch gekennzeichnet, dass** der mindestens eine Mikrowellenresonator einen Spaltsensor aufweist.

**8.** Verfahren nach einem der Ansprüche 1 bis 7, **dadurch gekennzeichnet, dass** die Messung an dem einlagigen Tabakpapier erfolgt.

**9.** Verfahren nach einem der Ansprüche 1 bis 8, **dadurch gekennzeichnet, dass** die Messung an dem zu einer Bobine aufgewickelten Tabakpapier erfolgt.

**10.** Verfahren nach einem der Ansprüche 1 bis 9, **dadurch gekennzeichnet, dass** die Messung in oder direkt nach dem Trockner erfolgt.

**11.** Verfahren nach einem der Ansprüche 1 bis 10, **dadurch gekennzeichnet, dass** die Messung vor einer Crimp-Einrichtung erfolgt.

**12.** Verfahren nach einem der Ansprüche 1 bis 11, **dadurch gekennzeichnet, dass** die Zugabe von Wasser und/oder Glycerin zu der breiigen Masse abhängig von mindestens einem der dichteunabhängigen Feuchtewerte erfolgt.

**13.** Verfahren nach einem der Ansprüche 1 bis 12, **dadurch gekennzeichnet, dass** als Feuchtewert jeweils ein Feuchtewert ($\Phi$) vorgesehen ist, der als Quotient aus der Verbreiterung der Halbwertsbreite (B) und der Resonanzfrequenzverschiebung (A) bestimmt wird, wobei jeweils leerer und befüllter Resonator miteinander verglichen werden.

**14.** Verfahren nach einem der Ansprüche 1 bis 12, **dadurch gekennzeichnet, dass** als Feuchtewert jeweils ein Feuchtewert ($\Phi$) vorgesehen ist, der sich als Arcus-Tangens des Quotienten aus der Verbreitung der Halbwertsbreite (B) und der Resonanzfrequenzverschiebung (A) ergibt.

**Claims**

**1.** Method for measuring the additive content in tobacco paper for electric cigarettes, which is produced from a pulpy mass of additives, water, flavorings, and tobacco, wherein the pulpy mass is dried to form a single-layer tobacco paper, **characterized in that** the tobacco paper is measured with at least one microwave resonator with two resonance modes with two resonance frequencies ($f_L$, $f_H$) is measured, wherein the smaller of the two resonance frequencies ($f_L$) lies in the frequency range of less than 1 GHz and the larger of the two resonance frequencies lies in the microwave range of more than 2 GHz, a density-independent moisture value ($\Phi_{L,H}$) is calculated for each of the two resonance modes, and the glycerol content (g) is determined as a function of the two moisture values ($\Phi_{L,H}$).

**2.** Method according to claim 1, **characterized in that** the additive consists completely or partially of glycerin.

**3.** Method according to claim 1 or 2, **characterized in that** the additive content (g) depends linearly on both density-independent moisture values ($\Phi_{L,H}$) and an offset value.

**4.** Method according to one of claims 1 to 3, **characterized in that** a moisture content for the tobacco paper is measured as a function of the density-independent moisture value ($\Phi_H$) at the higher frequency.

**5.** Method according to one of claims 1 to 4, **characterized in that** the additive content (g) is determined independently of the mass of the tobacco paper.

**6.** Method according to one of claims 1 to 5, **characterized in that** the at least one microwave resonator has a planar sensor.

**7.** Method according to one of claims 1 to 6, **characterized in that** the at least one microwave resonator has a gap sensor.

**8.** Method according to one of claims 1 to 7, **characterized in that** the measurement is performed on the single-layer tobacco paper.

**9.** Method according to one of claims 1 to 8, **characterized in that** the measurement is performed on the tobacco paper wound into a bobbin.

**10.** Method according to one of claims 1 to 9, **characterized in that** the measurement is performed in or directly after the dryer.

**11.** Method according to one of claims 1 to 10, **characterized in that** the measurement is carried out before a crimping device.

**12.** Method according to one of claims 1 to 11, **characterized in that** the addition of water and/or glycerin to the pulpy mass is carried out depending on at least one of the density-independent moisture values.

**13.** Method according to one of claims 1 to 12, **characterized in that** a moisture value ($\Phi$) is provided as the moisture value, which is determined as the quotient of the broadening of the half-value width (B) and the resonance frequency shift (A), wherein empty and filled resonators are compared with each other.

**14.** Method according to one of claims 1 to 12, **characterized in that** the moisture value is a moisture value ($\Phi$), which is the arctangent of the quotient of the spread of the half-value width (B) and the resonance frequency shift (A).

**Revendications**

**1.** Procédé de mesure de la teneur en additifs dans un papier enveloppe pour tabac pour cigarettes électriques, lequel est fabriqué à partir d'une masse pâteuse d'additifs, d'eau, d'arômes et de tabac, dans lequel la masse pâteuse est séchée en un papier enveloppe pour tabac à une seule couche, **caractérisé en ce que** le papier enveloppe pour tabac est mesuré avec au moins un résonateur à microondes présentant deux modes de résonance avec deux fréquences de résonance ($f_L$, $f_H$), dans lequel la plus petite des deux fréquences de résonance ($f_L$) se situe dans la plage de fréquence en dessous de 1 GHz et la plus grande des deux fréquences de résonance se situe dans la plage de microondes au-dessus de 2 GHz, une valeur d'humidité indépendante de la densité ($\Phi_{L,H}$) est calculée pour chacun des deux modes de résonance, la teneur en glycérine (g) est déterminée en fonction des deux valeurs d'humidité ($\Phi_{L,H}$).

**2.** Procédé selon la revendication 1, **caractérisé en ce que** l'additif est constitué entièrement ou partiellement de glycérine.

**3.** Procédé selon la revendication 1 ou 2, **caractérisé en ce que** la teneur en additifs (g) dépend linéairement des deux valeurs d'humidité indépendantes de la densité ($\Phi_{L,H}$) et d'une valeur de décalage.

**4.** Procédé selon l'une des revendications 1 à 3, **caractérisé en ce qu'**une humidité pour le papier enveloppe pour tabac est mesurée en fonction de la valeur d'humidité indépendante de la densité ($\Phi_H$) à la plus grande fréquence.

**5.** Procédé selon l'une des revendications 1 à 4, **caractérisé en ce que** la teneur en additifs (g) est déterminée indépendamment de la masse du papier enveloppe pour tabac.

**6.** Procédé selon l'une des revendications 1 à 5, **caractérisé en ce que** l'au moins un résonateur à microondes présente un capteur planaire.

**7.** Procédé selon l'une des revendications 1 à 6, **caractérisé en ce que** l'au moins un résonateur à microondes présente un capteur de fente.

**8.** Procédé selon l'une des revendications 1 à 7, **caractérisé en ce que** la mesure est effectuée sur le papier enveloppe pour tabac à une seule couche.

**9.** Procédé selon l'une des revendications 1 à 8, **caractérisé en ce que** la mesure est effectuée sur le papier enveloppe pour tabac enroulé en une bobine.

**10.** Procédé selon l'une des revendications 1 à 9, **caractérisé en ce que** la mesure est effectuée dans le sécheur ou directement après celui-ci.

**11.** Procédé selon l'une des revendications 1 à 10, **caractérisé en ce que** la mesure est effectuée en amont d'un dispositif de sertissage.

**12.** Procédé selon l'une des revendications 1 à 11, **caractérisé en ce que** l'ajout d'eau et/ou de glycérine à la masse pâteuse est effectué en fonction de l'une au moins des valeurs d'humidité indépendantes de la densité.

**13.** Procédé selon l'une des revendications 1 à 12, **caractérisé en ce qu'**une valeur d'humidité ($\Phi$) est prévue respectivement en tant que valeur d'humidité, laquelle est déterminée en tant que quotient de l'élargissement de la demi-largeur (B) et du décalage de fréquence de résonance (A), dans lequel le résonateur vide et le résonateur rempli sont comparés respectivement.

**14.** Procédé selon l'une des revendications 1 à 12, **caractérisé en ce qu'**une valeur d'humidité ($\Phi$) est prévue en tant que valeur d'humidité, laquelle est l'arc tangente du quotient de l'élargissement de la demi-largeur (B) et du décalage de fréquence de résonance (A).

MP

14

12

10

16

Fig. 1

Fig. 2

**0,9 GHz**

**5,6 GHz**

**Fig. 3**

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007041429 A1
- WO 2017080982 A1